# EUROPÄISCHE PATENTANMELDUNG

(11) **EP 1 679 092 A1**
(43) Veröffentlichungstag der Anmeldung: **12.07.2006**
(21) Anmeldenummer: 05011621.9
(22) Anmeldetag: 30.05.2005
(51) Int. Cl.: A61M 15/00, A61B 5/087

(54) **Inhalationsvorrichtung mit einem einen Arzneimittelvorrat simulierenden Widerstandskörper**

(30) Priorität: 10.01.2005 DE 102005001170
(71) Anmelder: PulmoTec GmbH, 89420 Höchstädt/Donau (DE)
(72) Erfinder: Pabst, Joachim, Dr., 64354 Reinheim (DE); Weuthen, Thomas, 89420 Höchstädt/Donau (DE)
(74) Vertreter: Manitz, Finsterwald & Partner GbR

(57) **Zusammenfassung**

Ein medizinisches Gerät weist ein Mundstück (12), einen Tablettenhalter (14) zur Aufnahme einer Tablette (16) mit Inhalationsmittel, eine Dosiereinrichtung und einen von einem Lufteinlass (20) zu einem Luftauslass (22) des Mundstück reichenden Luftkanal auf. Zur Freigabe einer Arzneimitteldosis ist eine atemzuggesteuerte Auslöseeinrichtung vorgesehen und in dem Luftkanal befindet sich ein Widerstandskörper (24).

## Beschreibung

Die vorliegende Erfindung betrifft ein medizinisches Gerät umfassend ein Mundstück, einen von einem Lufteinlass des Gerätes zu einem Luftauslass des Mundstücks reichenden Luftkanal, einen im Bereich des Luftkanals vorgesehenen Arzneimittelvorrat mit Inhalationsmittel, und eine Dosiereinrichtung. Ein solches Inhalationsgerät ist aus der EP 0 642 366 B1 bekannt.

Es ist ebenfalls bekannt, in Inhalationsgeräten eine atemzuggesteuerte Auslösung vorzusehen, um zu vermeiden, dass der Bediener die Freigabe einer Arzneimitteldosis und den Inhalationsvorgang miteinander koordinieren muss, was zu Schwierigkeiten führen kann.

Es ist die Aufgabe der vorliegenden Erfindung, ein medizinisches Gerät der eingangs genannten Art derart weiterzubilden, dass mit einfachen Mitteln eine Entscheidung dahingehend getroffen werden kann, ob die das medizinische Gerät benutzende Testperson eine Aspirationsfähigkeit besitzt, die für ein Benutzen des Gerätes ausreichend ist.

Die Lösung dieser Aufgabe erfolgt durch die Merkmale des Anspruchs 1 und insbesondere dadurch, dass das medizinische Gerät eine atemzuggesteuerte Auslöseeinrichtung aufweist, die durch ausreichendes Saugen an dem Mundstück eine Freigabe einer Arzneimitteldosis auslöst, und dass statt des Arzneimittelvorrats in dem Luftkanal ein den Luftwiderstand des Arzneimittelvorrats simulierender Widerstandskörper vorgesehen ist.

Erfindungsgemäß wird bei dem medizinischen Gerät der eingangs genannten Art kein Arzneimittelvorrat mit Inhalationsmittel vorgesehen. Stattdessen wird in den Luftkanal zwischen Lufteinlass und Luftauslass des Mundstücks zumindest ein Widerstandskörper vorgesehen, der den Luftwiderstand des Arzneimittelvorrats simuliert.

Aufgrund der atemzuggesteuerten Auslöseeinrichtung eine Freigabe einer Arzneimitteldosis alleine durch ausreichendes Saugen an dem Mundstück erreicht werden, das heißt durch Inhalieren von Luft, die durch den Luftkanal vom Lufteinlass bis zum Luftauslass des Mundstücks strömt. Um feststellen zu können, ob eine Testperson bzw. ein Patient eine ausreichende Aspirationsfähigkeit besitzt, um das medizinische Gerät, wenn es mit einem Arzneimittelvorrat mit Inhalationsmittel versehen ist, benutzen zu können, ist erfindungsgemäß kein Arzneimittelvorrat vorgesehen. Stattdessen ist in dem Luftkanal zwischen Lufteinlass und Luftauslass zumindest ein Widerstandskörper vorgesehen, der den Luftwiderstand des Arzneimittelvorrats simuliert, d.h. der den gleichen Luftwiderstand wie der Arzneimittelvorrat hervorruft. Hierdurch ist sichergestellt, dass innerhalb des Luftkanals die gleichen Strömungsverhältnisse vorliegen, wie wenn der Arzneimittelvorrat vorgesehen wäre. Auf diese Weise kann auf einfache Weise durch Saugen am Mundstück bzw. durch Inhalieren getestet werden, ob die atemzuggesteuerte Auslöseeinrichtung auslöst und somit ein ordnungsgemäßes Benutzen des medizinischen Gerätes möglich wäre.

Da in dem medizinischen Gerät nach der Erfindung kein Arzneimittelvorrat vorgesehen ist, kann das medizinische Gerät sehr kostengünstig hergestellt werden und es ist keine medizinische Zulassung erforderlich, die selbst dann notwendig wäre, wenn es sich bei dem Arzneimittelvorrat nur um ein Placebo handeln würde.

Das erfindungsgemäße medizinische Gerät kann sehr vorteilhaft in Arztpraxen oder Kliniken verwendet werden, um auf einfache Weise festzustellen, ob eine Testperson im Stande ist, das medizinische Gerät zu benutzen bzw. auszulösen. Gleichzeitig lässt sich das medizinische Gerät für Trainingszwecke einsetzen, um den Gebrauch des Pulverinhalators zu trainieren, ohne dass dabei ein Inhalieren von Inhalationsmittel und insbesondere von Arzneistoffen erfolgt.

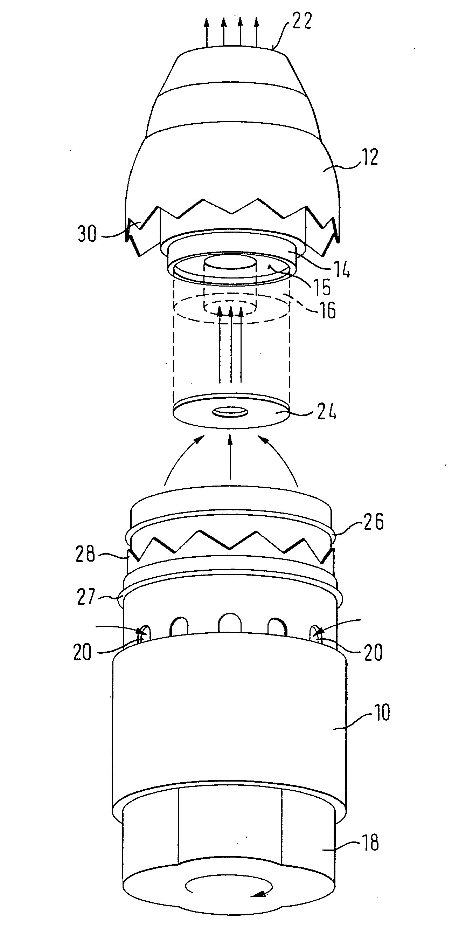

Vorteilhafte Ausführungsformen der Erfindung sind in der Beschreibung, der Zeichnung sowie den Unteransprüchen beschrieben.

Grundsätzlich kann der Arzneimittelvorrat in beliebiger Form vorgesehen werden, beispielsweise in Form von losem Pulver, Kartuschen oder dergleichen. Besonders vorteilhaft ist es, wenn der Arzneimittelvorrat in Form einer gepressten Tablette vorliegt. Hierbei kann zur Aufnahme der Tablette in dem Inhalationsgerät ein Tablettenhalter vorgesehen sein. In diesem Fall ist es vorteilhaft, wenn anstelle der Tablette der Widerstandskörper an dem Tablettenhalter angebracht wird.

Die Dosiereinrichtung kann ebenfalls grundsätzlich beliebig ausgebildet sein. Wenn als Arzneimittelvorrat eine Tablette verwendet ist, weist die Dosiereinrichtung vorzugsweise eine Abtrageinrichtung zum Abtragen von Inhalationsmittel von der Tablette auf.

Der den Luftwiderstand des Arzneimittelvorrats simulierende Widerstandskörper kann grundsätzlich an beliebigen Stellen innerhalb des Luftkanals vorgesehen sein, wobei der Widerstandskörper als separates Bauteil oder einstückig mit anderen Bauteilen des medizinischen Gerätes ausgebildet sein kann. Für eine besonders einfache Herstellung kann es vorteilhaft sein, den Widerstandskörper an einem Tablettenhalter und insbesondere an derjenigen Stelle des Tablettenhalters anzubringen, die an sich für die Aufnahme einer Tablette gedacht ist.

Nach einer weiteren vorteilhaften Ausführungsform ist der Widerstandskörper eine Lochblende, die so dimensioniert ist, dass sich gleiche Luftwiderstandsverhältnisse ergeben, unabhängig davon, ob ein Arzneimittelvorrat oder der Widerstandskörper in dem medizinischen Gerät vorgesehen ist.

Nach einer weiteren vorteilhaften Ausführungsform kann an dem Mundstück ein Saugrohr in Form eines Wegwerfartikels angebracht sein, beispielsweise ein Papp- oder Kunststoffröhrchen. Auf diese Weise lässt sich das medizinische Gerät für verschiedene Testpersonen einsetzen, ohne dass es erneut desinfiziert bzw. gereinigt werden muss.

Nach einem weiteren Aspekt der Erfindung betrifft diese ein Verfahren zum Ermitteln der Aspirationsfähigkeit einer Testperson unter Verwendung eines medizinischen Gerätes der oben beschriebenen Art, wobei von der Testperson an dem Mundstück gesaugt wird und in Abhängigkeit davon, ob eine Freigabe einer Arzneimitteldosis mit Hilfe der Auslöseeinrichtung ausgelöst wurde, die Aspirationsfähigkeit als ausreichend oder nicht ausreichend für die Benutzung des medizinischen Gerätes beurteilt wird.

Nachfolgend wird die vorliegende Erfindung rein beispielhaft anhand vorteilhafter Ausführungsformen und unter Bezugnahme auf die beigefügte Zeichnung beschrieben, wobei die Erfindung rein exemplarisch anhand eines Ausführungsbeispiels beschrieben wird, bei dem der Arzneimittelvorrat in Form einer Tablette in einem Pulverinhalator vorgesehen ist.

Die einzige Figur zeigt eine Explosionsansicht eines medizinischen Gerätes.

Das in der Figur dargestellte medizinische Inhalationsgerät umfasst eine Antriebseinheit 10 und ein auf die Antriebseinheit 10 aufsetzbares Mundstück 12, in dem ein Tablettenhalter 14 vorgesehen ist, an dessen Unterseite 15 normalerweise eine Ringtablette 16 angebracht ist, die in der Figur gestrichelt dargestellt ist.

In der Antriebseinheit 10 ist eine in der Figur nicht dargestellte Abtrageinrichtung in Form einer Stirnfräse vorgesehen, die von einem innerhalb der Antriebseinrichtung 10 befindlichen Antrieb (nicht dargestellt) antreibbar ist. Der Antrieb weist ein durch ein an der Unterseite der Antriebseinheit vorgesehenes Aufzugrad 18 spannbares Federwerk auf, welches die Stirnfräse nach einem entsprechenden Auslösen des Federwerks in Drehung versetzt, so dass von der in der Figur nach unten weisenden Stirnseite der Ringtablette 16 mit Arzneistoffen versehenes Inhalationsmittel abgerieben werden und eine Freigabe einer Arzneimitteldosis erfolgen kann, wenn das Mundstück 12 auf die Antriebseinheit 10 aufgesetzt ist. Hierbei wird der Tablettenhalter 14 mit einer Feder in Richtung der Stirnfräse gedrückt.

Bei aufgesetztem Mundstück 12 wird durch Saugen an dem Mundstück 12 ein mit Pfeilen angedeuteter Luftstrom von Lufteinlassöffnungen 20, die am Außenumfang der Antriebseinheit 10 vorgesehen sind, durch innerhalb der Antriebseinheit 10 vorgesehene Luftkanäle zum Außenumfang der Stirnfräse geleitet, wobei sich die Luftkanäle innerhalb der Stirnfräse fortsetzen, so dass die angesaugte Luft durch den Innenkanal der Ringtablette 16 und von dort zum Luftauslass 22 des Mundstücks 12 gelangen kann. Das durch Drehen der Stirnfräse von der Ringtablette 16 abgelöste Inhalationsmittel wird durch die angesaugte Luft, die durch den Luftkanal strömt, mitgerissen, so dass dieses anschließend inhaliert werden kann.

Im Inneren der Antriebseinheit 10 ist eine nicht dargestellte Auslöseeinrichtung vorgesehen, die atemzuggesteuert ist und die durch ausreichendes Saugen an dem Mundstück 12 eine Betätigung des zuvor mit Hilfe des Aufzugrades 18 gespannten Federwerks auslöst, so dass sich dieses dreht und dadurch die Stirnfräse antreibt. Die Auslöseeinrichtung kann dabei beispielsweise eine schwenkbare Klappe umfassen, die innerhalb des Luftkanals angeordnet ist und die ein Auslösen des Federwerks bewirkt, wenn eine ausreichende Luftmenge durch den Luftkanal strömt.

Gemäß der Erfindung ist an dem Tablettenhalter 15 keine Ringtablette 16 befestigt, sondern eine den Luftwiderstand der Tablette 16 simulierender Widerstandskörper 24, der beim dargestellten Ausführungsbeispiel die Form einer Lochblende besitzt, das heißt einer dünnen Scheibe, die mit einer mittigen Bohrung versehen ist. Hierbei ist der Querschnitt der Bohrung etwas kleiner als der Innenquerschnitt der Ringtablette 16 gewählt, so dass im Ergebnis das Inhalationsgerät den gleichen Strömungswiderstand innerhalb des Luftkanals aufweist, wie wenn statt des Widerstandskörpers 24 die Ringtablette 16 an dem Tablettenhalter 14 befestigt wäre.

Der Widerstandskörper 24 kann auf gleiche Weise wie die Ringtablette 16 an dem Tablettenhalter 15 befestigt werden, das heißt durch Festklemmen, Verkleben oder dergleichen. Anschließend kann das Mundstück 12 wie üblich auf die Antriebseinheit 10 aufgesetzt und mit Hilfe eines Schnappwulstes 26 verrastet werden. Durch eine unterhalb des Schnappwulstes 26 am Außenumfang der Antriebseinheit 10 vorgesehene Schnappverzahnung 28 und eine hierzu korrespondierende Schnappverzahnung 30, die am Mundstück 12 vorgesehen ist, ist eine Verdrehsicherung geschaffen. Das Bezugszeichen 27 bezeichnet eine Schnappwulst für eine Schutzkappe.

Um Feststellen zu können, ob eine Testperson bzw. ein Patient eine Aspirationsfähigkeit besitzt, die zur Bedienung des oben beschriebenen Inhalationsgerätes ausreicht, kann dieses auf einfache Weise statt mit einer Ringtablette 16 mit dem Widerstandskörper 24 versehen werden. Nach Spannen des Federwerks durch Drehen des Aufzugrades 18 kann die Testperson auf übliche Weise das Mundstück 12 in den Mund nehmen und inhalieren. Wenn anschließend die atemzuggesteuerte Auslöseeinrichtung auslöst, so dass sich das Federwerk hörbar entspannt, kann davon ausgegangen werden, dass die Aspiration der Testperson für einen ordnungsgemäßen Betrieb der Inhalationsvorrichtung ausreicht und einem Volumenstrom von etwa 40 1/min entspricht. Wenn die Testperson eine Auslösung des Antriebs - eine richtige Atemtechnik unterstellt - nicht bewirken kann, so ist das Inhalationsgerät für diese Testperson nicht geeignet.

Es sei darauf hingewiesen, dass eine Stirnfräse der oben beschriebenen Art in der deutschen Patentanmeldung 101 14 967 und dem deutschen Gebrauchsmuster 295 01 527 beschrieben ist. Die Offenbarung dieser beiden Schriften wird hiermit durch Bezugnahme ausdrücklich zum Gegenstand der vorliegenden Offenbarung gemacht.

### Bezugszeichenliste

- 10: Antriebseinheit
- 12: Mundstück
- 14: Tablettenhalter
- 15: Unterseite des Tablettenhalters
- 16: Ringtablette
- 18: Aufzugrad
- 20: Lufteinlassöffnungen
- 22: Luftauslass
- 24: Widerstandskörper
- 26: Schnappwulst für Mundstück
- 27: Schnappwulst für Schutzkappe
- 28, 30: Schnappverzahnung

## Patentansprüche

1. Medizinisches Gerät, umfassend ein Mundstück (12), einen von einem Lufteinlass (20) des Gerätes zu einem Luftauslass (22) des Mundstücks (12) reichenden Luftkanal, einen im Bereich des Luftkanals vorgesehenen Arzneimittelvorrat (16) mit Inhalationsmittel, und eine Dosiereinrichtung,
wobei
eine atemzuggesteuerte Auslöseeinrichtung vorgesehen ist, die durch ausreichendes Saugen an dem Mundstück (12) eine Freigabe einer Arzneimitteldosis auslöst, und
statt des Arzneimittelvorrats (16) zumindest ein den Luftwiderstand des Arzneimittelvorrats (16) simulierender Widerstandskörper (24) vorgesehen ist.

2. Vorrichtung nach Anspruch 1,
**dadurchgekennzeichnet**, **dass**
der Arzneimittelvorrat eine Tablette (16) ist.

3. Vorrichtung nach Anspruch 2,
**dadurchgekennzeichnet**, **dass**
zur Aufnahme der Tablette (16) ein Tablettenhalter (14) vorgesehen ist.

4. Vorrichtung nach einem der vorstehenden Ansprüche,
**dadurchgekennzeichnet**, **dass**
der Widerstandskörper (24) an einem Tablettenhalter (14) angebracht ist.

5. Vorrichtung nach einem der vorstehenden Ansprüche,
**dadurchgekennzeichnet**, **dass**
die Dosiereinrichtung eine Abtrageinrichtung zum Abtragen von Inhalationsmittel von einer Tablette (16) aufweist.

6. Vorrichtung nach einem der vorstehenden Ansprüche,
**dadurchgekennzeichnet**, **dass**
der Widerstandskörper eine Lochblende (24) ist.

7. Vorrichtung nach einem der vorstehenden Ansprüche,
**dadurchgekennzeichnet**, **dass**
an dem Mundstück (12) ein Saugrohr in Form eines Wegwerfartikels angebracht ist.

8. Verfahren zum Ermitteln der Aspirationsfähigkeit einer Testperson unter Verwendung eines medizinisches Gerätes, umfassend ein Mundstück, einen von einem Lufteinlass des Gerätes zu einem Luftauslass des Mundstücks reichenden Luftkanal, einen im Bereich des Luftkanals vorgesehenen Arzneimittelvorrat mit Inhalationsmittel, und eine Dosiereinrichtung, wobei eine atemzuggesteuerte Auslöseeinrichtung vorgesehen ist, die durch ausreichendes Saugen an dem Mundstück eine Freigabe einer Arzneimitteldosis auslöst, und
wobei statt des Arzneimittelvorrats zumindest ein den Luftwiderstand des Arzneimittelvorrats simulierender Widerstandskörper vorgesehen ist, bei welchem Verfahren von der Testperson an dem Mundstück gesaugt wird und in Abhängigkeit davon, ob eine Betätigung des Antriebs ausgelöst wurde oder nicht, die Aspirationsfähigkeit als ausreichend oder nicht ausreichend für die Benutzung des medizinischen Gerätes beurteilt wird.

9. Verfahren nach Anspruch 8,
**dadurchgekennzeichnet**, **dass**
an dem Mundstück (12) vor dem Saugen ein Saugrohr in Form eines Wegwerfartikels angebracht wird.

10. Verfahren nach Anspruch 8 oder 9,
**dadurchgekennzeichnet**, **dass**
als Arzneimittelvorrat eine Tablette verwendet wird.

11. Verfahren nach Anspruch 8, 9 oder 10,
**dadurchgekennzeichnet**, **dass**
als Widerstandskörper eine Lochblende verwendet wird.
